(19) Europäisches Patentamt
European Patent Office
Office européen des brevets

(11) Veröffentlichungsnummer: **0 004 881**
**B1**

(12) # EUROPÄISCHE PATENTSCHRIFT

(45) Veröffentlichungstag der Patentschrift: **07.10.81**

(21) Anmeldenummer: **79100928.5**

(22) Anmeldetag: **28.03.79**

(51) Int. Cl.³: **C 07 C 47/52,** C 07 C 47/228, C 07 C 45/29, B 01 J 35/00 // C07C95/08, C07C47/54

(54) **Verfahren zur Herstellung von aromatischen und araliphatischen Aldehyden.**

(30) Priorität: **21.04.78 DE 2817496**

(43) Veröffentlichungstag der Anmeldung: **31.10.79 Patentblatt 79/22**

(45) Bekanntmachung des Hinweises auf die Patenterteilung: **07.10.81 Patentblatt 81/40**

(84) Benannte Vertragsstaaten: **BE CH DE FR GB NL**

(56) Entgegenhaltungen:

DE-A-2 137 938

HOUBEN-WEYL: «Methoden der Organischen Chemie» 4. Auflage, Band VII, 1954, Teil 1: «Sauerstoffverbindungen» Georg Thieme Verlag Stuttgart (DE) Seiten 159-191

(73) Patentinhaber: **BASF Aktiengesellschaft, Carl-Bosch-Strasse 38, D-6700 Ludwigshafen (DE)**

(72) Erfinder: **Sauer, Wolfgang, Dr., Kaefertaler Strasse 183, D-6800 Mannheim 1 (DE)**
Erfinder: **Fliege, Werner, Dr., Authariestrasse 6, D-6701 Otterstadt (DE)**
Erfinder: **Dudeck, Christian, Dr., Odenwaldring 20, D-6703 Limburgerhof (DE)**
Erfinder: **Petri, Norbert, Dr., Max-Beckmann-Strasse 17, D-6710 Frankenthal (DE)**

EP 0 004 881 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von aromatischen und araliphatischen Aldehyden

Die Erfindung betrifft ein Verfahren zur Herstellung von aromatischen und araliphatischen Aldehyden durch Oxidation von entsprechenden Alkoholen mit Sauerstoff in Gegenwart einer Silberkatalysators bestimmter Korngrösse und unter bestimmten Bedingungen der Temperatur.

Es ist aus Houben-Weyl, Methoden der Organischen Chemie, Band 7/1, Seiten 159 bis 191, bekannt, dass Alkohole mit guten Ergebnissen nur dann zu den entsprechenden Aldehyden umgesetzt werden, wenn je nach Struktur des Alkohols spezifische Oxidationsmittel bzw. Katalysatoren und Bedingungen gewählt werden. So werden z.B. zur Herstellung von aliphatischen Aldehyden vorteilhaft Chromsäure (loc. cit., Seite 171), zur Herstellung von aromatischen Aldehyden wie Benzaldehyd Selendioxid (loc. cit., Seite 179) oder Salpetersäure empfohlen. Entsprechend werden auch bei der katalystischen Dehydrierung von Alkoholen im Falle von Methanol in erster Linie Kupfer-, Silber- und Zinkverbindungen genannt (loc. cit., Seite 160). Houben-Weyl gibt an (loc. cit., Seite 164), dass «bei den höheren Alkoholen (bereits von $C_8$ ab) ein merkliches Absinken der Aldehydausbeute eintritt, wahrscheinlich infolge Verteerung des Kontaktes». Es wird weiterhin darauf hingewiesen, dass Benzylalkohole schwerer als aliphatische Alkohole dehydrierbar sind; sie neigen leicht zur Ätherbildung und die entsprechenden aromatischen Aldehyde spalten leichter Kohlenmonoxid ab. Daher wird empfohlen, im Vakuum an reinen Kupfer- oder Silbermetallkontakten zu dehydrieren, wobei man «unter Berücksichtigung aller Vorsichtsmassnahmen bei 300°C die Benzaldehyde mit Ausbeuten bis zu 80% der Theorie» erhält (loc. cit., Seite 166). Zur Herstellung von Silberkontakten verwendet man Schwammsilber bzw. reduziert Silberoxid bei 400 bis 500°C mit Wasserstoff (loc. cit., Seite 162). Für die Dehydrierung von Phenyläthylalkohol wird als Katalysator Zinkoxid auf Bimsstein empfohlen, das bei 430°C eine Ausbeute von nur 50 Prozent liefert.

Als Reaktionstemperaturen im Falle der Dehydrierung von Benzylakohol an Kupferkatalysatoren nennt Ullmanns Encyklopädie der technischen Chemie, 4. Auflage, Band 8, Seite 436, ebenfalls 200 bis 300°C und gibt an, dass bei 380°C ausserdem Kohlenmonoxid, Kohlendioxid, Benzol und Toluol entstehen. Allgemein wird angegeben, dass technische Bedeutung für die Herstellung von Benzaldehyd nur die Oxidation von Toluol sowie die Seitenkettenchlorierung des Toluols zum Benzalchlorid mit anschliessender Verseifung erlangt haben (Ullmann, loc. cit., Seiten 344 bis 350).

Die DE-A-2137938 beschreibt einen Reaktor zur Herstellung von Aldehyden, insbesondere Formaldehyd durch oxidative Dehydrierung von Alkoholen unter Verwendung von Silber-Katalysatoren, wobei der Silberkatalysator sich über einem perforierten Kontaktboden befindet, dadurch gekennzeichnet, dass auf dem Kontaktboden eine, zur Aufnahme des Katalysators dienende, vom Boden beabstandete und sich stellenweise auf ihm abstützende gasdurchlässige Unterlage angeordnet ist. Zwischen der gasdurchlässigen Unterlage und dem Kontaktboden sind maximale Abstände von 1 cm, vorzugsweise von 2 bis 7 mm, vorgesehen. Als gasdurchlässige Unterlage ist eine geschmeidige Metallunterlage vorgesehen. Vorteilhaft ist die gasdurchlässige Unterlage als plissiertes oder gewelltes Gewebe ausgebildet und das Gewebe auf seiner Unterseite mit warzenförmigen Vorsprüngen versehen.

Man leitet Alkohol, Luft und gegebenenfalls Wasserdampf durch eine relativ dünne Schicht eines Silberkatalysators.

Die Unterlage muss so ausgebildet sein, dass sie sich dem Kontaktboden ohne Faltenbildung gut anpasst. Im Hinblick darauf wird eine geschmeidige Metallunterlage verwendet, die beispielsweise aus einem Chrom-Nickel-Stahl besteht. Die Bindungsart der Metallunterlage kann als Leinenbindung, Köperbindung oder als anderer Metalltuchgewebetyp ausgeführt sein.

Im Beispiel wird ein V2A-Netz mit einer Maschenweite von 0,5 mm mit einer wellenförmigen Plissierung beschrieben. Die Wellenberge wiesen einen Abstand von 10 mm und eine Höhe von 5 mm auf.

Die Umsetzung kann für den Fall der Verwendung von Benzylalkohol durch die folgenden Formeln wiedergegeben werden:

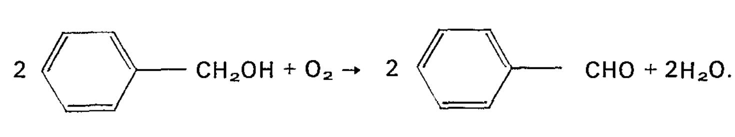

Im Vergleich zu den bekannten Verfahren liefert das Verfahren nach der Erfindung überraschend auf einfacherem und wirtschaftlicherem Wege ein besseres Gesamtergebnis mit Bezug auf Ausbeute und Reinheit der aromatischen und araliphatischen Aldehyde sowie Lebensdauer des Katalysators. Die Raum-Zeit-Ausbeute ist vergleichsweise besser. Eine umständliche Katalysatorherstellung bzw. Katalysatoraufbereitung wird vermieden. Unumgesetzter Alkohol kann bei der Destillation des Reaktionsgemisches abgetrennt und wieder in die Synthese zurückgeführt werden. Das erfindungsgemässe Verfahren gibt im Hinblick auf die bekannten Verfahren die Möglichkeit, eine Vielzahl von verschieden substituierten, aromatischen bzw. araliphatischen Aldehyden ohne apparative

Veränderungen nach einer Arbeitsweise in einer Anlage herzustellen. Das verwendete Oxidationsmittel Luft ist billig, leicht zu handhaben und bereitet im Gegensatz zu anderen Oxidationsmitteln keine Entsorgungsprobleme.

Im Hinblick auf die bekannten Verfahren besitzt der erfindungsgemässe Katalysator eine deutlich höhere Lebensdauer und kann auf einfacherem und wirtschaftlicherem Wege erhalten werden. Silberkristalle aller Teilchengrössen, wie sie auch bei der elektrolytischen Herstellung des Silbergranulats anfallen, können verwendet werden. Bei dem erfindungsgemässen Verfahren werden somit die Elektrolysieranlagen besser ausgenutzt und können entsprechend dimensioniert werden; Energie, Betriebspersonal und Hilfsstoffe, z.B. Salpetersäure, werden eingespart und Operationen wie Wäsche, Siebung und Trocknung des Silbers vereinfacht. Bei den bekannten Verfahren muss man das Silber auf den Träger erst noch aufbringen oder das Silber nachträglich dosieren. Alle diese vorteilhaften Ergebnisse des erfindungsgemässen Verfahrens sind überraschend. Man konnte im Hinblick auf den Stand der Technik nicht vermuten, dass durch Verwendung von reinen Silberkristallen besonderer Korngrösse anstatt Schaumsilber oder Silber auf Trägern oder anderen Katalysatoren eine Steigerung der Reaktionsgeschwindigkeit und damit der Raum-Zeit-Ausbeute, und zwar gerade bei wesentlich höheren Temperaturen, möglich ist. Die vorteilhaften Ergebnisse sind weiterhin insbesondere im Hinblick auf die Lehre von Houben-Weyl überraschend, denn man hätte angesichts der hohen erfindungsgemässen Temperaturen zumindest eine wesentliche Verschlechterung der Ausbeute und erhebliche Bildung von Zersetzungsprodukten erwarten sollen. Die aufwendige Arbeitsweise unter Vakuum und mit spezieller Katalysatorbehandlung wird vermieden. Hohe spezifische Katalysatorbelastungen, z.B. 0,1 bis 3 t/m² Katalysatorquerschnitt mal Stunde, können erzielt werden.

Im Hinblick auf das in DE-A-2137938 beschriebene Verfahren konnte nicht erwartet werden, dass die erfindungsgemässen Bedingungen gerade bei araliphatischen Alkoholen, noch dazu ohne die Notwendigkeit von entsprechend konstruierten Unterlagen, weit höhere Ausbeuten an Endstoff in dazu noch höher konzentrierten Endlösungen erhalten lassen. Dass die erfindungsgemässe Kombination, gerade auch die kurze Verweilzeit zusammen mit bestimmten Schichtverteilungen und Korngrössen der Silberteilchen, diese vorteilhaften Ergebnisse liefert, war überraschend. Dass insbesondere Teilchen von 0,1 bis unterhalb 0,4 mm dabei eine Rolle spielen, war im Hinblick auf die DE-A unerwartet. Überraschend sind diese Ergebnisse auch durch die Bedeutungslosigkeit von Unterlagen; alle Beispiele zeigen bei fehlenden Unterlagen nach DE-A hohe Ausbeuten und Katalysatoraktivität.

Als aromatische und araliphatische Aldehyde kommen zweckmässig solche Endstoffe der Formel

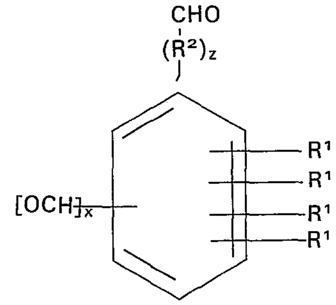

(I)

und dementsprechend als araliphatische Alkohole solche Ausgangsstoffe der Formel

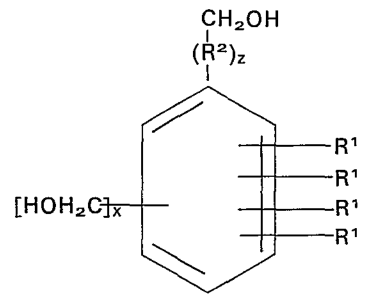

(II)

in betracht, worin die einzelnen Reste $R^1$ gleich oder verschieden sein können und jeweils ein Wasserstoffatom, einen aliphatischen Rest, den Rest $R^3$-O- oder den Rest

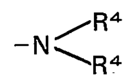

bedeuten, $R^2$ einen aliphatischen Rest bezeichnet, $R^3$ für ein Wasserstoffatom oder einen aliphatischen Rest steht, x und z gleich oder verschieden sein können und jeweils 0 oder 1 bedeuten, die einzelnen Reste $R^4$ gleich oder verschieden sein können und jeweils einen aliphatischen Rest bedeuten. Bevorzugte Ausgangsstoffe II und dementsprechend bevorzugte Endstoffe I sind solche, in deren Formeln die einzelnen Reste $R^1$ gleich oder verschieden sein können und jeweils ein Wasserstoffatom oder einen Alkylrest mit 1 bis 5 Kohlenstoffatomen, den Rest $R^3$-O- oder den Rest

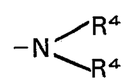

bedeuten, $R^2$ einen Alkylenrest mit 1 bis 5 Kohlenstoffatomen bezeichnet, $R^3$ für ein Wasserstoffatom oder einen Alkylrest mit 1 bis 5 Kohlenstoffatomen steht, die einzelnen Reste $R^4$ gleich oder verschieden sein können und jeweils einen Alkylrest mit 1 bis 5 Kohlenstoffatomen bedeuten, x und z gleich oder verschieden sein können und jeweils 0 oder 1 bedeuten. Da die Reaktion in der Gasphase durchgeführt wird, verwendet man im allgemeinen Alkohole II, die ohne Zersetzung leicht verdampfbar sind. Die vorgenannten Reste können noch durch unter den Reaktionsbedingungen

inerte Gruppen, z.B. Alkylgruppen mit 1 bis 4 Kohlenstoffatomen, substituiert sein. Als aromatische Aldehyde werden hier solche, in deren Formeln z=0 und somit $(R^2)_z$ eine Einfachbindung bedeuten, definiert, als araliphatische Aldehyde solche, in deren Formeln z=1 bezeichnet, definiert; in beiden Fällen sind die Ausgangsstoffe II araliphatische Alkohole. Man verwendet vorteilhaft Monoalkohole (x=0) oder Dialkohole (x=1) als Ausgangsstoffe II.

Es kommen z.B. als Ausgangsstoffe II in Frage:
2-Methylbenzylalkohol,
3-Methylbenzylalkohol,
4-Methylbenzylalkohol,
2-Methoxybenzylalkohol,
3-Methoxybenzylalkohol,
4-Methoxybenzylalkohol,
2,3-Dimethylbenzylalkohol,
3,4-Dimethylbenzylalkohol,
2,6-Dimethylbenzylalkohol,
3,5-Dimethylbenzylalkohol,
2,3-Dimethoxybenzylalkohol,
3,4-Dimethoxybenzylalkohol,
3,5-Dimethoxybenzylalkohol,
2-Äthylbenzylalkohol,
3-Äthylbenzylalkohol,
4-Äthylbenzylalkohol,
2,3-Diäthylbenzylalkohol,
3,4-Diäthylbenzylalkohol,
2,6-Diäthylbenzylalkohol,
3,5-Diäthylbenzylalkohol,
2-Äthoxybenzylalkohol,
3-Äthoxybenzylalkohol,
4-Äthoxybenzylalkohol,
2-n-Propylbenzylalkohol,
3-n-Propylbenzylalkohol,
4-n-Propylbenzylalkohol,
2,3-Di-n-propylbenzylalkohol,
3,4-Di-n-propylbenzylalkohol,
2,6-Di-n-propylbenzylalkohol,
3,5-Di-n-propylbenzylalkohol,
2-Isopropylbenzylalkohol,
3-Isopropylbenzylalkohol,
4-Isopropylbenzylalkohol,
2-Butylbenzylalkohol,
3-Butylbenzylalkohol,
4-Butylbenzylalkohol,
2-Isobutylbenzylalkohol,
3-Isobutylbenzylalkohol,
4-Isobutylbenzylalkohol,
2-tert.-Butylbenzylalkohol,
3-tert.-Butylbenzylalkohol,
4-tert.-Butylbenzylalkohol,
2,3-Diäthoxybenzylalkohol,
3,4-Diäthoxybenzylalkohol,
2,6-Diäthoxybenzylalkohol,
3,5-Diäthoxybenzylalkohol,
Benzylalkohol,
2,3,4-Trimethoxybenzylalkohol,
3,4,5-Trimethoxybenzylalkohol,
2,4,6-Trimethoxybenzylalkohol
und entsprechende durch die Äthyl-, n-Propyl-, Isopropyl-, n-Butyl-, sek.- Butyl-, Isobutyl-, tert.-Butyl-gruppe verätherte Trihydroxybenzylalkohole; unsubstituierte oder in vorgenannter Weise substituierte o-, m- oder p-Aminobenzylalkohole, deren Stickstoffatome durch die Methyl-, Äthyl-, n-Propyl-, Isopropyl-, n-Butyl-, sek.-Butyl-, Isobutyl-, tert.-Butyl-gruppe zweifach substituiert sind; o-, m- und p-Hydroxy-methylbenzylalkohol und entsprechende, in vorgenannter Weise am Kern substituierte Di-(hydroxymethyl)-benzole; unsubstituierte oder in vorgenannter Weise substituierte Phenyläthyl-, Phenylpropyl-, Phenylisopropyl-, Phenyl-butyl-, Phenyl-sek.-butyl-, Phenyl-tert.-butyl-, Phenyl- isobutyl-, Phenylpentyl-, Phenylhexyl-, Phenylisopentylalkohole.

Die Ausgangsstoffe II können auch in Mischung mit Wasser verwendet werden; die Konzentration der wässrigen Gemische kann zweckmässig zwischen 30 und 100 Gewichtsprozent, vorzugsweise zwischen 70 und 90 Gewichtsprozent Ausgangsstoff II schwanken. Der Ausgangsstoff II wird in Dampfform, vorteilhaft im Gemisch mit Wasserdampf und gegebenenfalls mit Inertgas, dem Reaktionsraum zugeführt. Als unter den Reaktionsbedingungen inerte Gase (Inertgas) werden zweckmässig Edelgase wie Xenon, Argon, Neon, Helium; Alkane wie Methan, Äthan, Propan, 2,2-Dimethylpropan, Butan, Pentan, Isobutan; Äther wie Dimethyläther, Methyläthyläther; bevorzugt Stickstoff, Kohlenmonoxid und/oder Kohlendioxid; und entsprechende Gemische verwendet. Das Inertgas kann für sich allein oder im Gemisch mit Wasserdampf und/oder dampfförmigem Ausgangsstoff II oder vorteilhaft im Gemisch mit Luft verwendet werden. Das Molverhältnis von Inertgas zu Sauerstoff beträgt insbesondere mindestens 4,4, zweckmässig 4,4 bis 20, vorteilhaft 6 bis 10 zu 1. Die Angaben bezüglich Inertgas beziehen sich hier stets auf die Gesamtmenge, d.h. einschliesslich des Inertgasanteils der bevorzugt verwendeten Luft.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Ausgangsstoff II werden zweckmässig im Molverhältnis von 0,25 bis 2,5, insbesondere von 0,5 bis 1,5 Mol Sauerstoff je Mol Ausgangsstoff II angewandt. Vorzugsweise beträgt die Gesamtmenge an Wasserdampf nicht mehr als 7, vorteilhaft von 1 bis 5 Mol je Mol Ausgangsstoff II. Die Luft und gegebenenfalls das Inertgas können direkt in die Verdampfung des Ausgangsstoffs II, zweckmässig in die siedende Ausgangsstoff II/Wasser-Mischung, oder an einer beliebigen Stelle vor dem Katalysator eingeleitet werden. Die Verweilzeit im Reaktionsraum beträgt vorteilhaft von 0,005 bis 0,5 zweckmässig von 0,01 bis 0,2, bevorzugt 0,01 bis 0,1 Sekunden.

Die Gesamtschichtdicke des Katalysators beträgt zweckmässig 10 bis 50, vorzugsweise 15 bis 40 Millimeter. Die Katalysatorteilchen in Gestalt von Silberkristallen befinden sich vorteilhaft im Katalysator des üblicherweise vertikal aufgestellten Reaktors in einer Schicht oder zweckmässiger je nach Korngrösse in einem oberen oder unteren Teil der Gesamtschicht oder vorzugsweise je nach

Korngrösse in einem oberen, mittleren oder unteren Teil der Gesamtschicht angeordnet. Das gesamte Katalysatorbett liegt zweckmässig auf einem Netz aus Silber oder vorgeglühtem Edelstahl. Bei grossen Reaktoren mit einem Durchmesser von mehr als 15 cm wird das Netz zweckmässig vor Einbau gewellt. Das Netz liegt vorteilhaft auf einem Lochboden. Unmittelbar unter dem Lochboden befindet sich zweckmässig ein Wasserkühler. Das Ausgangsgemisch aus dampfförmigem Ausgangsstoff II, Inertgas und Sauerstoff bzw. Luft und gegebenenfalls Wasserdampf wird im allgemeinen von oben nach unten geführt, so dass die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäss alle Angaben der Beschreibung über oberen (unteren) Teil des Katalysators für den entsprechenden, dem Ausgangsgemisch (dem abgeführten Reaktionsgemisch) zugewandten Teil, z.B. bei horizontal angeordneten Reaktoren für den vorderen (hinteren) Teil des Katalysators. Hat der Katalysator nur eine Schicht, so enthält diese Silberkristalle mit einer Korngrösse von 0,1 bis 2,5, vorteilhaft 0,1 bis 2, vorzugsweise 0,2 bis 1 Millimeter. Bei einem 2-Schichten-Katalysator enthält zweckmässig die obere Teilschicht 40 bis 80, vorzugsweise von 50 bis 75 Gewichtsprozent des Katalysators mit Teilchen der Korngrösse 0,1 bis 0,75 Millimeter, die untere Teilschicht 20 bis 60, vorzugsweise von 25 bis 50 Gewichtsprozent des Katalysators mit Teilchen der Korngrösse 0,75 bis 2,5, vorteilhaft von 0,75 bis 1 Millimeter.

Bei einem 3-Schichten-Katalysator befinden sich im unteren Teil zweckmässig 20 bis 40, vorzugsweise 25 bis 35 Gewichtsprozent aller Katalysatorteilchen, im mittleren Teil 40 bis 70, vorzugsweise 40 bis 60 Gewichtsprozent aller Katalysatorteilchen, im oberen Teil 10 bis 30, vorzugsweise 15 bis 25 Gewichtsprozent aller Katalysatorteilchen. Die Teilchen des unteren Schichtteils haben Korngrössen von 0,75 bis 2,5, vorzugsweise 0,75 bis 1, die des mittleren Schichtteils von 0,4 bis 0,75, die des oberen Schichtteils 0,1 bis 0,4 Millimeter.

Zweckmässig belastet man den Katalysator mit 0,1 bis 3 t, insbesondere 0,2 bis 1,5 t dampfförmigem Ausgangsstoff II je $m^2$ Katalysatorbettquerschnitt und Stunde. Zur grosstechnischen Ausführung verwendet man bevorzugt Katalysatorbettdurchmesser von mindestens 0,05, zweckmässig 0,1 bis 3 Metern. Die Umsetzung wird zweckmässig bei einer Temperatur von 450 bis 700°C, vorzugsweise bei einer Temperatur von 475 bis 650°C, insbesondere 500 bis 625°C, drucklos oder unter Druck, kontinuierlich durchgeführt. Man kann in Abwesenheit zusätzlicher Lösungsmittel umsetzen, man kann aber auch unter den Reaktionsbedingungen inerte, organische Lösungsmittel verwenden. Als Lösungsmittel kommen z.B. in Frage: aromatische Kohlenwasserstoffe, z.B. Toluol, Benzol, Äthylbenzol, o-, m-, p-Xylol, Isopropylbenzol, Methylnapthalin; Äther, z.B. Äthylpropyläther, Methyltert.-butyläther, n-Butyläthyläther, Di-n-butyläther, Diisobutyläther, Diisoamyläther, Diisopropyläther, Cyclohexylmethyläther, Diäthyläther, Äthylenglykoldimethyläther, Tetrahydrofuran, Dioxan; Ketone wie Methyläthylketon, Aceton, Diisopropylketon, Diäthylketon, Methylisobutylketon, Mesityloxid, Acetophenon, Cyclohexanon, Äthylisoamylketon, Diisobutylketon, Methylcyclohexanon, Dimethylcyclohexanon; aliphatische oder cycloaliphatische Kohlenwasserstoffe, z.B. Heptan, Pinan, Nonan, Benzinfraktionen innerhalb eines Siedepunktintervalls von 70 bis 190°C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; und entsprechende Gemische. Zweckmässig verwendet man das Lösungsmittel in einer Menge von 50 bis 1000 Gewichtsprozent, vorzugsweise von 100 bis 300 Gewichtsprozent, bezogen auf Ausgangsstoff II.

Die Oxidation kann wie folgt durchgeführt werden: In ein Verdampfungsaggregat, z.B. einen Fallstromverdampfer, werden einzeln oder im Gemisch Ausgangsstoff II und gegebenenfalls Wasser und/oder organisches Lösungsmittel eingegeben und verdampft, zweckmässig bei 70 bis 280°C. Anschliessend wird das Gasgemisch aus dampfförmigem Ausgangsstoff II, Luft, gegebenenfalls Inertgas und Wasserdampf in vorgenannten Mengen bei der Reaktionstemperatur durch den Katalysator geleitet. Das Verfahren wird im allgemeinen bei Drücken zwischen 0,5 und 3 bar, vorzugsweise zwischen 0,8 und 1,8 bar, kontinuierlich durchgeführt. Zweckmässig wird vor Beginn des Verfahrens der Silberkatalysator auf eine Temperatur von 250 bis 500, vorzugsweise von 380 bis 450°C erhitzt. Der Beginn der exothermen Umsetzung wird zweckmässig festgestellt, indem man Luft dem Ausgangsgemisch zugibt und die Temperaturveränderung im Katalysator prüft. Setzt die Reaktion ein, beobachtet man sofort einen Anstieg der Temperatur, andernfalls wird die Temperatur durch die Zuführung der kalten Luft sinken. Die Temperatur wird zweckmässig im Katalysator durch Thermoelemente gemessen. Ab Beginn der Reaktion leitet man im allgemeinen die Luft kontinuierlich dem dampfförmigen Ausgangsgemisch zu, gegebenenfalls unter Einleiten durch den Sumpf des Verdampfungsaggregates. Es ist vorteilhaft, die die Katalysatorzone verlassenden Reaktionsgase innerhalb kurzer Zeit abzukühlen, z.B. auf Temperaturen von 20 bis 160°C. Der Hauptteil des Endstoffes wird so kondensiert. Das abgekühlte Gasgemisch wird dann zweckmässig einem Absorptionsturm zugeführt, in welchem der Endstoff mit einem geeigneten Lösungsmittel wie Dimethylformamid, Dimethylsulfoxid, Aceton, Methanol oder Wasser sowie deren Gemische und/oder in vorgelegtem Kondensat früherer Umsetzungen, vorteilhaft im Gegenstrom, aus dem Gasgemisch gewaschen wird. Aus Kondensat und den Absorbaten wird der Endstoff I dann in üblicher Weise, z.B. durch Destillation, isoliert.

Die nach dem Verfahren der Erfindung herstellbaren aromatischen und araliphatischen Aldehyde sind wertvolle Ausgangsstoffe für die Herstellung

von Farbstoffen, Schädlingsbekämpfungsmitteln, Kunststoffen, Riechstoffen. Bezüglich der Verwendung wird auf die vorgenannten Veröffentlichungen verwiesen.

Die in den folgenden Beispielen angegebenen Teile sind Gewichtsteile.

*Beispiel 1:*

Man verwendet eine Anlage mit Verdampfer und einem senkrechten Rohrreaktor. Der Reaktor enthält an seinem Kopf die Zuführung für das dampfförmige Ausgangsgemisch und die Reaktorhaube. Die Katalysatorschicht liegt unterhalb des Reaktorkopfes, weiter unten folgt eine Kühlzone. Der Reaktor ist mit 4 Absorptionskolonnen verbunden.

In den Reaktor wird ein Katalysator aus Silberkristallen (84 Teilen) folgender Zusammensetzung eingetragen:

| | Anteil am Katalysator (Gew.%) | Korngröße mm |
|---|---|---|
| Schicht 1 | 20 | 0,1 -0,4 |
| Schicht 2 | 50 | 0,4 -0,75 |
| Schicht 3 | 30 | 0,75-1 |

Die Höhe des Katalysators beträgt 30 mm. Dem Verdampfer wird pro Stunde ein Gemisch von 250 Teilen Benzylalkohol, 100 Teilen Wasser und 210 Teilen Luft zugeführt und verdampft. Das dampfförmige Ausgangsgemisch wird durch den Katalysator geleitet und bei 600°C und 1,1 bar umgesetzt. Die Verweilzeit beträgt 0,055 Sekunden, die Katalysatorbelastung 0,36 t/m². h. Das Reaktionsgemisch wird nun auf 30°C abgekühlt. In Form einer 86-gewichtsprozentigen Lösung erhält man in den vereinigten organischen Phasen des Kondensats und der mit Wasser betriebenen Absorptionskolonnen 208 Teile pro Stunde Benzaldehyd, entsprechend einer Ausbeute von 85% der Theorie, bezogen auf verwendeten Ausgangsstoff II. Der Umsatz beträgt 98 Prozent, die Raum-Zeit-Ausbeute 10 g Benzaldehyd pro cm³ Katalysatorvolumen und Stunde.

*Beispiel 2:*

Es wird die gleiche Anlage wie in Beispiel 1 verwendet. In den Reaktor wird ein Katalysator aus Silberkristallen (84 Teilen) folgender Zusammensetzung eingetragen:

| | Anteil am Katalysator (Gew.%) | Korngröße mm |
|---|---|---|
| Schicht 1 | 65 | 0,1 -0,75 |
| Schicht 2 | 35 | 0,75-1 |

Die Höhe des Katalysators beträgt 30 mm. Analog Beispiel 1 wird stündlich ein Gemisch aus 560 Teilen p-tert.-Butylbenzylalkohol und 120 Teilen Wasser mit 270 Teilen Luft sowie 380 Teilen Stickstoff als Inertgas bei 520°C und 1,1 bar über den Katalysator geführt. Die Verweilzeit beträgt 0,025 Sekunden, die Katalysatorbelastung 0,8 t/m². h.

Analog Beispiel 1 erhält man in Form einer 90-gewichtsprozentigen organischen Phase 398 Teile pro Stunde p-tert.-Butylbenzaldehyd, entsprechend einer Ausbeute von 72% der Theorie. Der Umsatz beträgt 98 Prozent, die Raum-Zeit-Ausbeute 18 Gramm p-tert.-Butylbenzaldehyd pro cm³ Katalysatorvolumen und Stunde.

*Beispiel 3:*

Es wird die gleiche Anlage wie in Beispiel 1 verwendet. Analog Beispiel 1 wird stündlich ein Gemisch aus 900 Teilen p-Methylbenzylalkohol und 900 Teilen Aceton verdampft und mit 890 Teilen Luft und 200 Teilen Stickstoff als Inertgas bei 620°C und 1,2 bar über den Katalysator geführt. Die Verweilzeit beträgt 0,015 Sekunden, die Katalysatorbelastung 1,3 t/m². h. Die Umsetzung wird analog Beispiel 1 durchgeführt. In Form einer 43-gewichtsprozentigen Lösung erhält man 666 Teile pro Stunde p-Tolylaldehyd, entsprechend einer Ausbeute von 75% der Theorie. Der Umsatz beträgt 99 Prozent, die Raum-Zeit-Ausbeute 31 Gramm pro cm³ Katalysatorvolumen und Stunde.

*Beispiel 4:*

Es wird die gleiche Anlage wie in Beispiel 1 verwendet. Analog Beispiel 1 wird stündlich ein Gemisch aus 320 Teilen m-Dimethylaminobenzylalkohol und 50 Teilen Wasser mit 300 Teilen Luft bei 530°C und 1,2 bar über den Katalysator geführt. Die Verweilzeit beträgt 0,035 Sekunden, die Katalysatorbelastung 0,45 t/m². h. In Form einer 88-gewichtsprozentigen organischen Phase erhält man 228 Teile pro Stunde m-Dimethylaminobenzaldehyd, entsprechend einer Ausbeute von 72% der Theorie. Der Umsatz beträgt 94 Prozent, die Raum-Zeit-Ausbeute 11 Gramm m-Dimethylaminobenzaldehyd pro cm³ Katalysatorvolumen und Stunde.

*Beispiel 5:*

Es wird die gleiche Anlage wie in Beispiel 1 verwendet. In den Reaktor wird ein Katalysator aus Silberkristallen (82 Teilen) der Korngrössen von 0,1 bis 2,5 mm in einer homogenen Schicht eingetragen. Die Höhe des Katalysators beträgt 30 mm. Analog Beispiel 1 wird stündlich ein Gemisch aus 150 Teilen 2-Phenyläthanol und 60 Teilen Wasser mit 210 Teilen Luft bei 540°C und 1,1 bar über den Katalysator geführt. Die Verweilzeit beträgt 0,07 Sekunden, die Katalysatorbelastung 0,2 t/m². h. In Form einer 73-gewichtsprozentigen organischen Phase erhält man 99 Teile pro Stunde Phenylacetaldehyd, entsprechend einer Ausbeute von 67% der Theorie. Der Umsatz beträgt 78 Prozent, die Raum-Zeit-Ausrobeute 5 Gramm Phenylacetaldehyd pro cm³ Katalysatorvolumen und Stunde.

*Beispiel 6:*

Analog Beispiel 1 wird stündlich ein Gemisch aus 150 Teilen 2-Phenyl-1-propanol mit 60 Teilen Stickstoff und 185 Teilen Luft bei 550°C und 1,1 bar über den Katalysator geführt. Die Verweilzeit beträgt 0,08 Sekunden, die Katalysatorbelastung 0,2 t/m². h. In Form einer 78-gewichtsprozentigen organischen Phase erhält man 108 Teile pro Stunde 2-Phenylpropanal, entsprechend einer Ausbeute von 73% der Theorie. Der Umsatz beträgt 92 Prozent, die Raum-Zeit-Ausbeute 5 Gramm 2-Phenylpropanal pro cm³ Katalysatorvolumen und Stunde.

*Beispiel 7:*

Analog Beispiel 1 wird stündlich ein Gemisch aus 300 Teilen 3-(p-tert.-Butylphenyl)-2-methyl-1-propanol und 200 Teilen Wasser mit 250 Teilen Luft und 250 Teilen Stickstoff bei 560°C und 1,1 bar über den Katalysator geführt. Die Verweilzeit beträgt 0,025 Sekunden, die Katalysatorbelastung 0,42 t/m². h. In Form einer 77-gewichtsprozentigen organischen Phase erhält man 211 Teile pro Stunde 3-(p-tert.-Butylphenyl)-2-methylpropanal, entsprechend einer Ausbeute von 71% der Theorie. Der Umsatz beträgt 95 Prozent, die Raum-Zeit-Ausbeute 10 Gramm 3-(p-tert.-Butylphenyl)-2-methylpropanal pro cm³ Katalysatorvolumen und Stunde.

*Beispiel 8:*

Analog Beispiel 1 wird stündlich ein Gemisch aus 400 Teilen 3,4,5-Trimethoxybenzylalkohol und 400 Teilen Aceton mit 380 Teilen Luft bei 530°C und 1,2 bar über den Katalysator geführt. Die Verweilzeit beträgt 0,04 Sekunden, die Katalysatorbelastung 0,57 t/m². h. In Form einer 44-gewichtsprozentigen Lösung erhält man 302 Teile pro Stunde 3,4,5-Trimethoxybenzaldehyd, entsprechend einer Ausbeute von 76% der Theorie. Der Umsatz beträgt 95 Prozent, die Raum-Zeit-Ausbeute 14 Gramm 3,4,5-Trimethoxybenzaldehyd pro cm³ Katalysatorvolumen und Stunde.

*Beispiel 9:*

Analog Beispiel 1 wird stündlich ein Gemisch aus 200 Teilen 4-(Hydroxymethyl)-benzylalkohol und 400 Teilen Dioxan mit 290 Teilen Luft bei 600°C und 1,1 bar über den Katalysator geführt. Die Verweilzeit beträgt 0,05 Sekunden, die Katalysatorbelastung 0,3 t/m². h. In Form einer 22-gewichtsprozentigen Lösung erhält man 124 Teile pro Stunde Terephthaldialdehyd, entsprechend einer Ausbeute von 63% der Theorie. Der Umsatz beträgt 92 Prozent, die Raum-Zeit-Ausbeute 6 Gramm Terephtaldialdehyd pro cm³ Katalysatorvolumen und Stunde.

**Patentanspruch**

Verfahren zur kontinuierlichen Herstellung von aromatischen und araliphatischen Aldehyden durch Oxidation von araliphatischen Alkoholen in Gegenwart von Metallkatalysatoren bei erhöhter Temperatur, *dadurch gekennzeichnet*, dass man araliphatische Alkohole in Gegenwart von Silberkristallen mit einer Korngrösse von 0,1 bis 2,5 Millimetern bei einer Temperatur von 450 bis 700°C mit Sauerstoff im Molverhältnis von 0,25 bis 2,5 Mol Sauerstoff je Mol araliphatischen Alkohol und bei Drücken zwischen 0,5 und 3 bar mit einer Verweilzeit im Reaktionsraum von 0,005 bis 0,5 Sekunden oxidiert.

**Claim**

A process for the continuous preparation of aromatic and araliphatic aldehydes by oxidizing araliphatic alcohols in the presence of a metal catalyst at an elevated temperature, *characterized in that* an araliphatic alcohol is oxidized with oxygen at a temperature of from 450 to 700°C and a pressure of from 0.5 to 3 bar in a ratio of from 0.25 to 2.5 moles of oxygen per mole of araliphatic alcohol in the presence of silver crystals having a particle size of from 0.1 to 2.5 millimeters, the residence time in the reaction chamber being from 0.005 to 0.5 second.

**Revendication**

Procédé pour la préparation en continu d'aldéhydes aromatiques et araliphatiques par oxydation d'alcools araliphatiques à température élevée en présence de catalyseurs métalliques, caractérisé en ce que l'oxydation des alcools araliphatiques par l'oxygène est réalisée en présence de cristaux d'argent d'une granulométrie de 0,1 à 2,5 mm à une température comprise entre 450 et 700°C et sous une pression comprise entre 0,5 et 3 bars, les proportions molaires d'oxygène et d'alcool étant dans un rapport de 0,25 à 2,50 moles d'oxygène par mole d'alcool araliphatique et la durée du séjour dans la zone de réaction étant de 0,005 à 0,5 seconde.